(19) Europäisches Patentamt
European Patent Office
Office européen des brevets

(11) EP 0 552 770 B1

(12) **EUROPEAN PATENT SPECIFICATION**

(45) Date of publication and mention of the grant of the patent:
**16.07.2003 Bulletin 2003/29**

(51) Int Cl.[7]: **G06K 9/46**, A61B 5/117

(21) Application number: **93100900.5**

(22) Date of filing: **21.01.1993**

(54) **Apparatus for extracting facial image characteristic points**

Vorrichtung zum Extrahieren von Merkmalen eines Gesichtsbildes

Dispositif pour extraire des points caractéristiques d'une image faciale

(84) Designated Contracting States:
**DE FR GB**

(30) Priority: **23.01.1992 JP 975392**

(43) Date of publication of application:
**28.07.1993 Bulletin 1993/30**

(73) Proprietor: **MATSUSHITA ELECTRIC INDUSTRIAL CO., LTD.**
**Kadoma-shi, Osaka 571-8501 (JP)**

(72) Inventors:
- **Kado, Yoshiyasu**
  **Kadoma City, 571 (JP)**
- **Nakagawa, Masamichi**
  **Hirakata City, 573 (JP)**
- **Maehara, Fumio**
  **Moriguchi City, 570 (JP)**

(74) Representative: **Schirdewahn, Jürgen et al**
**Jung Schirdewahn Lemke**
**Clemensstrasse 30**
**80803 München (DE)**

(56) References cited:
- **PROCEEDINGS OF THE 1989 IEEE INTERNATIONAL SYMPOSIUM ON CIRCUITS AND SYSTEMS, CAT. NO. 89CH2692-2, vol. 2 , 11 May 1989 , PORTLAND, OREGON, USA pages 1362 - 1365 XP000131701 ALATTAR A.M. AND RAJALA S.A. 'PRIMITIVE-BASED IMAGE CODING TECHNIQUE FOR STILL PICTURES'**
- **JOURNAL OF COMPUTER-ASSISTED MICROSCOPY (USA) vol. 2, no. 4 , December 1990 pages 239 - 247 RUSS J.C. 'Direction Information from the Sobel Operator'**
- **NTZ ARCHIV vol. 8, no. 9 , October 1986 , BERLIN DE BUHR R. 'FRONT FACE ANALYSIS AND CLASSIFICATION (Analyse und Klassification von Gesichtsbildern)'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### 1. FIELD OF THE INVENTION

The present invention relates to a characteristic points extraction part of an individual person identification apparatus and an facial shape characteristics. It is exemplified by a facial expression recognition apparatus for the facial image picture communication.

### 2. DESCRIPTION OF THE RELATED ART

Heretofore, as the method through which characteristic points or characteristic quantities are extracted from a facial image picture, there were, for example, Japanese unexamined Patent Publication (Tokkai) JP-61-208185 (208185/1986) and Japanese unexamined Patent Publication (Tokkai) JP-63-223974 (223974/1988). The former is such that a facial image picture inputted from a image picture input part is once stored in an image picture memory part, then it is binary-leveled in a binary level conversion part with an appropriate threshold value θ. Then the characteristic regions of a human face are extracted using characteristic parameters (area circumferential length, coordinate of the center of gravity, etc.) from this binary-leveled image picture. The latter is such that the tint conversion is applied to a facial image picture stored in a image picture memory part, and then the obtained skin-color region is taken as a mask pattern and the characteristic regions are extracted.

In the above-mentioned prior art, in case that a binary-leveled image picture is used, when the threshold value θ varies, areas of obtained characteristic regions vary also. Hence there was a drawback that the characteristic points exhibited deviations depending upon the threshold value θ. Moreover, even for the same face, when the position of lighting source differs at the time of inputting the image picture, the brightness distribution on the face becomes different. Hence when the binary level conversion is done with a certain fixed threshold value of θ, areas of characteristic regions change. This causes the consequence that different extraction results are issued from a single same face.

Furthermore, when the hue is used as has been described above, there has been a problem that, depending on the sort of lighting sources, such as sun light, fluorescent lamp and others, the hue of respective regions including the skin-color regions change. Because of these, in the prior art, it was necessary to fix the position of lighting, colors, and others.

Also, in case that the hue information is used,

if a television camera used as for the image picture input part, at the time of A/D-converting and storing the input signal into an image picture memory part, the hue information becomes unstable in such regions that is including sharp edges, making an accurate extraction of the characteristic points impossible.

An apparatus of the kind defined by the precharacterizing features of claim 1 is known from "Proceedings of the 1989 IEEE International Symposium on Circuits and Systems, Cat. No. 89CH2692-2, vol. 2, 11 May 1989, Portland, Oregon, USA, pp. 1362-1365, Alatta R A.M. & Rajala S.A., "Primitive-based image coding technique for still pictures".

An apparatus for extracting facial image characteristic points based on an image segmentation process including an edge extraction part form performing edge-stressing process on edge parts included in said facial image data to make an edged-image data and a binary level conversion part for performing conversion of the edged-image data into binary-leveled edged-image data on each preestimated region of facial elements is known from "NTZ Archiv vol. 8, no. 9, October 1986, Berlin, DE, Buhr R., pp. 245-256, "Front Face Analysis and Classification (Analyse und Klassification von Gesichtsbildern).

### OBJECT AND SUMMARY OF THE INVENTION

According to the present invention, in order to solve the above-mentioned problem, an apparatus is provided as defined by the features of claim 1. Advantageous developments of the invention are defined by the subclaims.

In the present invention, for the facial image picture inputted from an image picture input part, an edged image picture is produced by the edge extraction part. The edged image picture includes a large amount of minute noise due to such as moustache or wrinkles. Therefore, for a searching region,i.e., a region to be searched in this image picture, the above-mentioned edged image picture thus obtained by the edge extraction part is converted into a binary-leveled edged image picture by a binary level conversion part. From the searching regions of obtained binary-leveled edged image picture, such a region that is close to the shape data stored in a shape data-base part is selected based on the magnitude of their correspondence factor obtained by an image picture arithmetic processing part. The shape data are updated in a manner that the correspondence factor becomes large in the vicinity of selected region by a shape data updating part. Then, when the correspondence factor outputted from the image picture arithmetic processing part reaches a certain value or more based on those updated shape data, the characteristic points that is the object of the search are outputted from the output part.

The apparatus of the present invention, owing to the binary level conversion of the edged image picture, is robust against the conditions for taking pictures, such as position of lighting source, color, and others. And since the shape data are stored as the data-base, even for the facial image picture wearing the glasses, for example, erroneous action of the apparatus becomes seldom. Furthermore, owing to the inclusion of the shape data updating part in the apparatus, personal difference depending on individual persons can be absorbed, enabling us to raise the capability of the characteristic points extraction.

While the novel features of the invention are set forth particularly in the appended claims, the invention, both as to organization and content, will be better understood and appreciated, along with other objects and features thereof, from the following detailed description taken in conjunction with the drawings.

## BRIEF DESCRIPTION OF THE DRAWINGS

FIG.1 is a drawing of constitution of a first embodiment of the present invention.
FIG.2 shows an example of the input image picture and its searching regions.
FIG.3 shows an example of the shape data in the shape data-base part.
FIG.4 shows an example of shapes of respective facial elements.
FIG.5 shows a constitutional drawing of one embodiment of the present invention stated in claim 2.
FIG.6 shows a constitutional drawing of one embodiment of the present invention stated in claim 4.
FIG.7 shows a constitutional drawing of one embodiment of the present invention stated in claim 6.
FIG.8 shows a conventional drawing representing an example of hardware configurations of the present invention.
FIG.9(a) and FIG.9(b) in combination show a flow chart of an example of the procedure of extraction of the facial image characteristic points in the present invention.

It will be recognized that some or all of the Figures are schematic representations for purposes of illustration and do not necessarily depict the actual relative sizes or locations of the elements shown.

## DESCRIPTION OF THE PREFERRED EMBODIMENT

In FIG.1, a constitutional drawing of a first embodiment of the present invention is shown. The output of the image picture input part 1 to which the facial images from a television camera or the likes are inputted is given to an edge extraction part 2, wherein the edge processing is applied to the inputted image picture. The output whereon the edge processing has been applied in the edge extraction part 2 is given to a binary level conversion part 3. In this part, for image picture data, binary level conversion process is performed for each preestimated region of respective facial elements. In a shape data-base part 4, shape data of facial elements such as iris, nose, mouth, and eyebrow are stored. Binary-leveled edged image picture, which is processed by the two-level conversion part 3, and the shape data from the shape data-base part 4 are inputted into the image picture arithmetic processing means 5, wherein the correspondence factor therebetween is computed. Starting from the correspondence factor thus obtained, contents of the shape data-base part 4 is updated in a manner that the corresponding factor increases by the shape data updating part 6. From the shape data of the shape data-base part 4 and the correspondence factor obtained by the image picture arithmetic processing part 5, the characteristic points of the image picture are extracted and issued from the output part 7.

In the following, explanation is given more to the details on the procedure of extracting the characteristic points.

First, the facial image picture is taken from the image picture input part 1, and an edged image picture is produced by the edge extraction part 2 from the inputted image picture. In this part, computing is made by using an operator such as, for example, the Sobel operator (see, for example, p.98 of D. H. Ballard and C. M. Brown, translated by Akio Soemura "Computer Vision", Japan Computer Association, 1987), wherefrom gradient vectors at respective pixels can be obtained. The gradient vectors thus obtained have their respective magnitudes as well as their directions. The direction of the gradient vector means a direction in which the gradient of brightness of the image picture takes a largest value, and the magnitude thereof means this largest value. Hereinafter, they are also called as the edge vectors, since those regions along such the pixels having large gradient vector magnitudes may form edges in the image picture. FIG.2 shows an example of the inputted image picture and the searching regions of respective facial elements.

In the following, taking the iris as an example, the procedure of extracting the characteristic points is described. First, the magnitudes m of edge vectors in a searching region of the iris shown in FIG.2 are converted into binary-leveled values of 0 or 1 using a certain threshold value $\theta$. That is, the raw gradient vectors obtained by applying a gradient operation, such as Sobel operation described above, on the brightness data of respective pixels (or positions), are normalized and converted into either of unit vectors or zero vectors. Hereinafter, for the convenience of explanation, sign of these unit vectors obtained as has been described above is reversed (multiplied by -1), and they are called again as the edge vectors (More accurately, they should be called as the normalized edge vector). This process is carried out in the binary level conversion part 3. Since the magnitudes m of edge vectors varies depending on the state of the lighting source used at the time of taking the image picture, the above-mentioned threshold value $\theta$ must be determined from the frequency distribution of the magnitude m. For example, the threshold value $\theta$ is determined in a manner that the binary level conversion is made by setting those data, which fall within 20 % probability of distribution from the largest magnitude in a relevant searching region, to be 1; whereas the rest those data falling within 80 % probability thereof to be 0.

In FIG.3 and FIG.4, an example of the contents stored in the shape data-base part 4 is shown. FIG.3 shows a shape data of an iris, as an example. In this case, number n of data-elements of the shape data is 12, that is, n=12. The shape data comprise 12 coordinate data and 12 gradient vectors at those respective coordinates. Coordinate data, $l_k$ and $m_k$ are coordinates at which gradient vectors, $v_x$ and $v_y$ are given. The gradient vector, $v_y$, are unit vectors giving the direction in which largest gradient value is present. Since the iris has a circular shape inside of which is much darker than the outside thereof, coordinate data form a circle and all the gradient vectors given at these coordinates direct to the center of the circle.

FIG.4 shows examples of facial elements and their shapes. Facial elements are iris, mouth, nose, eyebrow, and cheek, in this embodiment.

Next, the searching region for the edged image picture of iris is scanned and the correspondence factor $\phi$ between the inputted observed edge vectors and the gradient vectors of the shape data stored in the database is calculated in the arithmetic processing part 5. Since both of the inputted observed data and the shape data stored in the data-base part 4 are given in the form of vector, the correspondence factor $\phi$ to be required to calculate can be expressed by the average of inner products between those corresponding two vectors in a manner shown below.

Letting the inputted observed edge vectors in the edged image picture be

$$u_{i,j} = (u_x, u_y)$$

where i, j are x, y coordinates of positions on the image picture and

$$u_x^2 + u_y^2 = 1 \text{ or } 0,$$

and the shape data be

coordinate data:

$$p_k = (l_k, m_k)$$

where $l_k$, $m_k$ are x, y coordinates of positions whereat the shape data is given, respectively, and

gradient vectors:

$$v_k = (v_x, v_y)$$

where

$$u_x^2 + u_y^2 = 1$$

and

$$1 \leqq k \leqq n$$

(n is number of the positions),

then the correspondence factor $\phi$ of the shape data at coordinates (i,j) in the image picture can be expressed as

$$\phi = (\Sigma u_{i+l_k, j+m_k} \cdot v_k)/n,$$

where

$$1 \leqq k \leqq n$$

and

$$u_{i+l_k, j+m_k} \cdot v_k = u_x \cdot v_x + u_y \cdot v_y.$$

In a manner described as above and scanning the coordinate (i,j), the correspondence factor $\phi$ for respective coordinates (i,j) in the searching region is calculated. Among them, a plural number of coordinates at which values of the correspondence factor $\phi$ are large are assigned to be preestimated regions of the relevant facial element.

Next, in respective preestimated regions, the shape data are updated by the shape data updating part 6, and then the correspondence factor $\phi$ is again searched. The scheme of updating is, for example, to move the coordinate of one position of the present data by +1 and then -1 in the direction of the gradient vector and to take either one direction in which the correspondence factor increases. After this movement, the direction of the gradient vector is also updated in a manner that it coincides with the shape data. In such the manner, all of the elements of shape data are successively updated in a manner that the correspondence factor $\phi$ is further improved. When the improvement of the correspondence factor $\phi$ stops, or when $\phi$ exceeds a certain specified value s, any further updating of the shape data is stopped.

Thereafter, in accordance with the updated shape data, the characteristic points are issued from the output part 7. The scheme of this outputting is as follows: For example, when a final values of the correspondence factor $\phi$ is less than a certain value t (s>t), regarding the region in which the correspondence factor $\phi$ is maximum to be a preestimated region and taking the shape data there to be a facial element to seek, only the necessary characteristic points thereof are issued. And in case that there are a plural number of preestimated regions in which ϕ is larger than a certain value t, the preestimated region is determined by, for example, a statistical procedure. That is, those regions which are disposed mutually close are all regarded to be genuine shape data. And then, by calculating the average with regard to corresponding positions of all of these shape data, new shape data are obtained.

Then, taking the obtained shape data to be the shape data of a facial element, namely the object to search for, only the necessary characteristic points are outputted. For example, in the case of iris, the average of coordinates of all the positions of the shape data are calculated to be a center point, and the maximum point and minimum point in the y-coordinate are taken to be the top and the bottom points of the iris, and then resultant data are issued from an output part.

Hereupon, since two irises are present in the x-coordinate direction on a face, it is necessary to issue two positions separated by a distance d or more at which the correspondence factor ϕ is large to be the two irises.

In a similar manner, the respective characteristic points of mouth, nose, eyebrow, and cheek can be extracted. For example, for the mouth, five points of top, bottom, left, right, and center are extracted; and for eyebrow, four points of top, bottom, left, and right are extracted.

And, in FIG.5, the constitution of a second embodiment is shown. In FIG.2, first, the searching region is selected only to the iris region, for example. And then, the searching regions for remaining facial elements are determined by the region determination part 8 based on the extracted characteristic points of the irises. In accordance with such process, it becomes possible to extract the characteristic points for remaining facial elements with a lesser amount of calculation. For example, the determination of the searching regions for the remaining facial elements can be processed by utilizing simple common knowledge such that the nose is present between mouth and eyes, and eyebrows are present immediately above eyes.

Furthermore, once the coordinates of two irises are determined, any possible tilt angle of the inputted facial image picture can be obtained. Hence, based on this tilt angle, by reversely rotating the shape data stored in the shape data-base part 4 by an amount of this obtained tilt angle by the shape data updating part 6, even from a tilted facial image picture, the extraction of the characteristic points becomes possible.

In FIG.6, a constitutional drawing of a third embodiment of the present invention is shown. The eyebrow has an edge which is not sharp but gradual. This is because the borders of hair of the eyebrow are gradual. Therefore, differing from-other facial elements, for the eyebrow, it is difficult to obtain strong edge components. Consequently, for the extraction of the characteristic points of the eyebrow, by applying a preprocessing of binary level conversion on the searching regions of eyebrows by a binary level conversion part 11, it becomes possible to obtain strong edge components. This preprocessing is selected by the process selection part 10.

The application of the above capability of the present invention is not limited to the eyebrow, but also valid, for example, to such one as moustache wherein its edge component is also gradual. And, in particular, in case of extracting the characteristic points of the eyebrow, since the eyebrow is oblong horizontally, its brightness distribution differs largely between both ends. Consequently, if the searching region is binary-leveled at only one time, it can happen that an accurate shape of eyebrow does not appear. Then, (as in the aspect described in claim 5,) the searching region of the eyebrow is divided into small sub-regions in the vertical direction. In respective small sub-regions, respective threshold values for binary level conversion are respectively determined in a manner that j % probabilities of brightness distribution is set to 0. Hereupon, j is determined in accordance with, for example, the area of respective searching regions. By obtaining the average and variance of the threshold values for respective small sub-regions, respective regions can be binary-leveled individually. At this process, in case that the threshold value deviates largely from the average value, it is regarded to be either one of

(i) There is no eyebrow in that small sub-region, or

(ii) There exist a lot of hair. Thus the process is carried out on respective small sub-regions by which the whole parts of those small sub-regions are binary-leveled totally to 0 or 1 in accordance with the above classifications.

The present embodiment is valid not only for such the case that the lighting source deviates from the center of the face to the right or left direction, but also it is effective in that the influence of hair existing in the eyebrow region can be reduced.

In FIG.7, a constitutional drawing of a fourth embodiment of the present invention is shown. In the case of the present embodiment, the preestimated regions obtained for respective facial elements are recorded in the adoption/rejection part 9; and from the combination thereof, one giving an adequate facial shape is selected. As the condition to obtain an adequate facial shape, the following facts, for example, can be used:

(iii) Nose and mouth are present on an equidivision-perpendicular line between two irises,

(iv) Distance between two eyebrows and that between two irises are almost the same, and

(v) The right cheek and the left cheek are present at almost equidistant places to the right hand and the left hand from the above equidivision-perpendicular line.

In such the manner, the best fit preestimated regions are searched for respective facial elements. And then for respective preestimated regions, the characteristic points which are the object of search can be obtained based on the shape data.

In the above-mentioned example, explanation of the present invention has been given on four different embodiments. Such embodiments may be constituted by hard ware as shown in the Figures, but may be constituted also by data processor either. In order to elucidate the present invention embodied by using the data processor or a computer specifically and concretely, an example of hardware configuration of computer for use in the present invention is shown in FIG.8, and an example of the procedure of extraction of the facial image characteristic points, which has been already described in the above embodiments, is now explained using a flow chart shown in FIG.9(a) and FIG.9(b).

FIG.8, shows a circuit block diagram giving a fundamental hardware configuration of the apparatus of the present invention. The facial image picture is taken into the apparatus through a television camera 101. The facial image picture signal issued from the television camera 101 is inputted into an A/D converter 102. A central processing unit, CPU 103 executes all the required functions, such as data access, transfer, store, arithmetic processing, and other functions for data under instructions of program installed in the apparatus. Functions or parts represented by boxes in FIG.5 through FIG.7 are preferably executed by such the installed program. Numeral 104 designates an image picture memory. The output of the A/D converter is memorized through a CPU 103 in an input image picture memory 104A as the input image picture data for all of each pixel. The input image picture data are converted into edged image picture data and further converted binary-leveled edged image picture data through the CPU 103. They are stored in an edged image picture memory 104B and a binary-leveled edged image picture memory 104C, respectively. Numeral 105 is a memory for storing the shape data-base of facial elements such as eyes, mouth, eyebrow, or cheek. data-base of each facial element includes three different sizes of small, medium and large. The correspondence factor between the binary-leveled edged image picture data and the shape data stored in the shape data-base memory 105 is computed; and the shape data are updated in a manner that the correspondence factor increases by the CPU 103. Numeral 106 is a working area of memory used for temporary purpose of the processing. Numeral 107 is an output area of memory for storing the extracted facial image characteristic points of necessary facial elements.

In FIG.9(a) and FIG.9(b) in combination, a flow chart of an example of the procedure of extraction of the facial image characteristic points is shown. In FIG.9(a), a flow starting at a start 201 through a step 216 corresponds to the process for the extraction of facial image characteristic points of eyes, whereas in FIG.9(b), flow of a step 302 through a step 316 corresponds to the process for the extraction of facial image characteristic points of mouth. For remaining other facial elements, almost the same flow chart as for the above two facial elements can be applied.

Hereupon, in the present invention it is unnecessary to use any color picture image, and thereby the extraction of the characteristic points is possible even from a monochromatic photograph. For the shape data, by preparing a plural number of data for one facial element, the obtainable accuracy of extraction of the characteristic points can be improved.

Although the present invention has been described in terms of the presently preferred embodiments, it is to be understood that such disclosure is not to be interpreted as limiting. Various alterations and modifications will no doubt become apparent to those skilled in the art after having read the above disclosure. Accordingly, it is intended that the appended claims be interpreted as covering all alterations and modifications as fall within the scope of the invention.

## Claims

**1.** An apparatus for outputting coordinate data which indicate features of a face corresponding to facial element shape data stored in a shape data-base, said apparatus comprising:

an image picture inputter (1, 101, 102, 104 A) for receiving an input image of a face,

an edge extractor (2, 103, 104 B) for processing said input image of said face to form an edged-image of said face;

a binary level converter (3, 103, 104 C) for dividing said image into a plurality of facial feature searching regions each of which corresponds to a respective facial feature of said face and subsequently performing a binary level conversion on said edged-image of said face corresponding to a plurality of facial elements, said binary level converter converting said edged-image of said face into a binary-leveled edged-image of said face;

a shape data-base (4, 105) permanently storing a plurality of shape data as a reference data base for said plurality of facial elements;

an image picture arithmetic processor (5, 103, 106) for determining an amount of correspondence by computing a correlation value between data of each of said plurality of facial feature points of said binary-leveled edged-image of said face and each corresponding one of said plurality of facial elements of said shape data stored in said shape data-base (4);

**characterized by**:

a shape data changer (6, 103), for changing the shape data copied from the shape data-base (4) based on the correlation value;

wherein said shape data is subject to an iterative modification until there is no further improvement in the correlation value or until a special correspondence value threshold is attained, and

an outputter (7, 107) for issuing output data based on said modified shape data,

wherein said data stored in said shape database (4)comprise coordinate data and gradient vectors in x, y coordinates.

**2.** An apparatus in accordance with claim 1, said apparatus further comprising: a facial feature region determiner (8, 103) for determining locations of others of said plurality of facial feature searching regions based on said data output from said outputter (7, 107) with respect to a determination of a first facial feature.

**3.** An apparatus in accordance with claim 2, wherein:

a region of irises of said face is determined as a first facial feature before said facial feature region determiner determines said locations of said others of said plurality of facial feature searching regions.

**4.** An apparatus in accordance with claim 2, further comprising a second binary level converter (11, 103) arranged to perform a binary level conversion before said processing of said input image of said face by said edge extractor (2, 103, 104 B).

**5.** An apparatus in accordance with claim 4, wherein said second binary level converter (11, 103) is connected to said edge extractor (2, 103, 104 B) and is arranged to receive an output of said facial feature region determiner, to divide one of said plurality of facial feature searching regions into a plurality of smaller sub-regions, and to determine a binary level conversion threshold value for each of said plurality of smaller sub-regions from a brightness distribution of each of said plurality of smaller sub-regions.

**6.** An apparatus in accordance with claim 1, said apparatus further comprising:

an adoption/rejection selector (9, 103) for obtaining expected positions of said plurality of facial features, which obtains and determines an accuracy of positions of said plurality of facial features, and which discards any erroneously extracted facial features prior to processing said input image of said face by said edge extractor (2, 103, 104 B).

**7.** An apparatus in accordance with claim 1, wherein:

said edge extractor (2, 103, 104 B) includes a computer using a Sobel operator which produces gradient vectors for respective pixels of said image of said face, said gradient vectors having magnitudes and directions indicative of said facial features.

**8.** An apparatus in accordance with claim 1, wherein:

an arithmetic computation performed by said image picture arithmetic processor (5, 103, 106) is an inner product between compared gradient vectors of said binary-levelled edged-image of said face and respective shape data.

**9.** An apparatus in accordance with claim 8, wherein:

a location of at least one of said plurality of facial feature searching regions is determined by a location on said image of said face where said correlation value is largest.

**10.** An apparatus in accordance with claim 3, wherein:

said respective locations of said others of said plurality of facial feature searching regions are determined with reference to a position of said region of said irises of said face.

**11.** An apparatus in accordance with claim 1, wherein said shape database changer (6, 103) changes said copied data by a method comprising:

(1) displacing said coordinate data by ±1 in a direction of said gradient vector,
(2) changing said direction of said gradient vector after said step of displacing said coordinate data, so as to match said shape data at said displaced coordinate data,
(3) repeating steps (1) and (2) by displacing said coordinate data further by ±1 thereby pursuing an increase in value of said correlation value, and
(4) ceasing said repeating step when one of said value of said correlation value no longer increases and said value of said correlation value exceeds a predetermined threshold value.

**12.** An apparatus in accordance with claim 1, wherein said shape data comprises:

said coordinate data which are defined as:

$$P_k = (l_k, m_k),$$

where $l_k$, $m_k$, are x, y coordinates of a k-th position at which said shape data is given, respectively, and
said gradient vectors are defined as:

$$v_k = (v_x, v_y),$$

where

$$v_x^2 + v_y^2 = 1$$

and

$$l<k<n$$

(where n is a number of positions).

**13.** An apparatus in accordance with claim 2, wherein said shape data comprises:

said coordinate data which are defined as:

$$P_k = (l_k, m_k),$$

where $l_k$, $m_k$, are x, y coordinates of a k-th position at which said shape data is given, respectively, and
said gradient vectors are defined as:

$$v_k = (v_x, v_y),$$

where

$$v_x^2 + v_y^2 = 1$$

and

$$l<k<n$$

(where n is a number of positions).

**Patentansprüche**

**1.** Vorrichtung zum Ausgeben von Koordinatendaten, die Merkmale eines Gesichts entsprechend Gesichtselementformdaten bezeichnen, die in einer Formdatenbasis gespeichert sind, wobei die Vorrichtung aufweist:

Eine Bildeingabeeinrichtung (1, 101, 102, 104A) zum Empfangen eines eingegebenen Gesichtsbilds;
eine Randextraktionseinrichtung (2, 103, 104B) zum Verarbeiten des eingegebenen Bilds des Gesichts zum Bilden eines geschärften Bilds des Gesichts;
eine Binärbewertungsumsetzeinrichtung (3, 103, 104C) zum Unterteilen des Bilds in mehrere Gesichtselementsuchbereiche, von denen jeder einem jeweiligen Gesichtsmerkmal des Gesichts entspricht und zum darauf folgenden Durchführen einer Binärbewertungsumsetzung bezüglich des geschärften Bilds des Gesichts entsprechend mehreren Gesichtselementen, wobei der Binärbewertungsumsetzer das geschärfte Bild des Gesichts in ein binär bewertetes geschärftes Bild des Gesichts umsetzt;
eine Formdatenbasis (4, 105), die mehrere Formdaten als Referenzdatenbasis für die mehreren Gesichtelemente dauerhaft speichert;
einen Bildrechenprozessor (5, 103, 106) zum Ermitteln eines Übereinstimmungsausmaßes durch Berechnen eines Korrelationswertes zwischen Daten von jedem der mehreren Gesichtsmerkmale des binär bewerteten geschärften Bilds des Gesichts und jeweils einem entsprechenden der mehreren Gesichtselement der Formdaten, die in der Formdatenbasis (4) gespeichert sind;

**gekennzeichnet durch**:

Eine Formdatenänderungseinrichtung (6, 103) zum Ändern der Formdaten, die aus der Formdatenbasis (4) kopiert sind auf Grundlage des Korrelationswertes;

wobei die Formdaten einer iterativen Modifikation unterworfen werden, bis keine weitere Verbesserung des Korrelationswertes vorliegt oder bis eine spezielle Übereinstimmungswertschwelle erzielt ist, und
eine Ausgabeeinrichtung (7, 107) zum Ausgeben von Ausgangsdaten auf Grundlage der modifizierten Formdaten,
wobei die in der Formdatenbasis (4) gespeicherten Daten Koordinatendaten und Gradientenvektoren in x-/y-Koordinaten umfassen.

**2.** Vorrichtung nach Anspruch 1, wobei die Vorrichtung außerdem aufweist: Eine Gesichtsmerkmalsbereichsermittlungseinrichtung (8, 103) zum Ermitteln von anderen Stellen als die mehrere Gesichtsmerkmalssuchbereiche auf Grundlage der Daten, die von der Ausgabeeinrichtung (7, 107) in Bezug auf eine Ermittlung eines ersten Gesichtsmerkmals ausgegeben werden.

**3.** Vorrichtung nach Anspruch 2, wobei ein Bereich der Iris des Gesichts (als erstes Gesichtsmerkmal) ermittelt wird, bevor die Gesichtsmerkmalsbereichsermittlungseinrichtung die Stellen der anderen der mehreren Gesichtsmerkmalssuchbereiche ermittelt.

**4.** Vorrichtung nach Anspruch 2, außerdem aufweisend eine zweite Binärbewertungsumsetzeinrichtung (11, 103), die dazu dient, eine Binärbewertungsumsetzung vor einer Verarbeitung des eingegebenen Bilds des Gesichts durch die Randextraktionseinrichtung (2, 103, 104B) durchzuführen.

**5.** Vorrichtung nach Anspruch 4, wobei die zweite Binärbewertungsumsetzeinrichtung (11, 103) mit der Randextraktionseinrichtung (2, 103, 104B) verbunden und dazu ausgelegt ist, ein Ausgangssignal von der Gesichtsmerkmalsbereichsermittlungseinrichtung zu empfangen, um einen der mehreren Gesichtsmerkmalssuchbereiche in mehrere kleiner Teilbereiche zu unterteilen, und um einen Binärbewertungsumsetzschwellenwert für jeden der mehreren kleinen Teilbereiche aus einer Helligkeitsverteilung von jedem der mehreren kleineren Teilbereiche zu ermitteln.

**6.** Vorrichtung nach Anspruch 1, wobei die Vorrichtung außerdem aufweist:

Eine Anpassungs-/Abweisungswahleinrichtung (9, 103) zum Gewinnen erwarteter Positionen der mehreren Gesichtsmerkmale,

wobei diese Einrichtung die Genauigkeit von Positionen der mehreren Gesichtsmerkmale gewinnt und ermittelt und sämtliche fehlerhaft extrahierte Gesichtsmerkmale vor einer Verarbeitung des eingegebenen Bilds des Gesichts durch die Randextraktionseinrichtung (2, 103, 104B) verwirft.

**7.** Vorrichtung nach Anspruch 1, wobei: Die Randextraktionseinrichtung (2, 103, 104B) einen Computer unter Verwendung eines Sobel-Operators enthält, der Gradientenvektoren für jeweilige Pixel des Bilds des Gesichts erzeugt, wobei die Gradientenvektoren Größen und Richtungen aufweisen, die die Gesichtsmerkmale anzeigen.

**8.** Vorrichtung nach Anspruch 1, wobei eine arithmetische Berechnung, durchgeführt durch den Bildrechenprozessor (5, 103, 106) ein inneres Produkt zwischen verglichenen Gradientenvektoren des binär bewerteten geschärften Bilds des Gesichts und jeweiligen Formdaten bildet.

**9.** Vorrichtung nach Anspruch 8, wobei: Eine Position von zumindest einem der mehreren Gesichtsmerkmalssuchbereiche durch eine Position auf dem Bild des Gesichts festgelegt ist, an der der Korrelationswert am größten ist.

**10.** Vorrichtung nach Anspruch 3, wobei die jeweiligen Positionen der übrigen der mehreren Gesichtsmerkmalssuchbereiche unter Bezug auf eine Position des Bereichs der Iris des Gesichts ermittelt werden.

**11.** Vorrichtung nach Anspruch 1, wobei die Formdatenbasisänderungseinrichtung (6, 103) die kopierten Daten durch ein Verfahren ändert, das folgende Schritte aufweist:

(1) Verschieben der Koordinatendaten um $\pm 1$ in einer Richtung des Gradientenvektors,
(2) Ändern der Richtung des Gradientenvektors nach dem Schritt der Verschiebung der Koordinatendaten, um die Formdaten an die verschobenen Koordinatendaten anzupassen,
(3) Wiederholen der Schritte (1) und (2) durch Verschieben der Koordinatendaten zusätzlich um $\pm 1$, wodurch eine Vergrößerung des Werts des Korrelationswerts erhalten wird, und
(4) Beenden des Wiederholungsschritts, wenn der Wert des Korrelationswerts nicht mehr größer wird und der Wert des Korrelationswerts einen vorbestimmten Schwellenwert übersteigt.

**12.** Vorrichtung nach Anspruch 1, wobei die Formdaten aufweisen:

Die Koordinatendaten, die definiert sind als:

$$P_k = (l_k, m_k),$$

wobei $l_k$, $m_k$ x-/y-Koordinaten einer k-ten Position sind, in der die Formdaten vorliegen, und die Gradientenvektoren definiert sind als:

$$v_k = (v_x, v_y),$$

wobei

$$v_x^2 + v_y^2 = 1$$

und

1<k<n

(wobei n die Anzahl der Positionen ist).

**13.** Vorrichtung nach Anspruch 2, wobei die Formdaten aufweisen:

Die Koordinaten, die definiert sind als:

$$P_k = (l_k, m_k),$$

wobei $l_k$, $m_k$ x-/y-Koordinaten einer k-ten Position sind, in der die Formdaten vorliegen, und die Gradientenvektoren definiert sind als:

$$v_k = (v_x, v_y),$$

wobei

$$v_x^2 + v_y^2 = 1$$

und

1<k<n

(wobei n die Anzahl der Positionen ist).

## Revendications

**1.** Un dispositif pour produire en sortie des données de coordonnées qui indiquent des caractéristiques d'un visage correspondant à des données de forme d'éléments faciaux mémorisées dans une base de données de formes, ledit dispositif comprenant :

un moyen d'entrée d'image (1, 101, 102, 104 A) pour recevoir une image d'entrée d'un visage,
un extracteur de bord (2, 103, 104 B) pour traiter ladite image d'entrée dudit visage afin de former une image bordée dudit visage ;
un convertisseur au niveau binaire (3, 103, 104 C) pour diviser ladite image en une pluralité de régions de recherche de caractéristique faciale dont chacune correspond à une caractéristique faciale respective dudit visage et ensuite exécuter une conversion au niveau binaire sur ladite image bordée dudit visage correspondant à une pluralité d'éléments faciaux, ledit convertisseur au niveau binaire convertissant ladite image bordée dudit visage en une image bordée convertie au niveau binaire dudit visage ;
une base de données de formes (4, 105) mémorisant de façon permanente une pluralité de données de formes en tant que base de données de référence pour ladite pluralité d'éléments faciaux ;
un processeur arithmétique d'image (5, 103, 106) pour déterminer la quantité de correspondance en calculant une valeur de corrélation entre des données de chacun de ladite pluralité de points de caractéristiques faciales de ladite image bordée convertie au niveau binaire et chaque élément facial correspondant de ladite pluralité d'éléments faciaux desdites données de formes mémorisées dans ladite base de données de formes (4) ;

**caractérisé par** :

un modificateur de données de forme (6, 103), pour modifier les données de forme copiées dans la base de données de formes (4) en fonction de la valeur de corrélation ;

dans lequel ladite donnée de forme est soumise à une modification itérative jusqu'à ce qu'aucune amélioration supplémentaire ne se produise pour la valeur de correction ou jusqu'à ce qu'un seuil de valeur de correspondance spécial soit atteint, et
un moyen de sortie (7, 107) pour délivrer des données de sortie en fonction de ladite donnée de forme modifiée,
dans lequel la donnée mémorisée dans ladite base de données de formes (4) comprend des données de coordonnées et des vecteurs gradients en coordonnées x, y.

**2.** Un dispositif selon la revendication 1, ledit dispositif comprenant en outre : un moyen de détermination dé région de caractéristique faciale (8, 103) pour déterminer des emplacements d'autres régions de ladite pluralité de régions de recherche de caractéristiques faciales en fonction de ladite donnée produite par ledit dispositif de sortie (7, 107) pour ce qui concerne une détermination d'une première caractéristique faciale.

**3.** Un dispositif selon la revendication 2, dans lequel :

une région d'iris dudit visage est déterminée comme étant une première caractéristique faciale avant que ledit moyen de détermination de région de caractéristique faciale détermine lesdits emplacements desdites autres régions de ladite pluralité de régions de recherche de caractéristique faciale.

**4.** Un dispositif selon la revendication 2, comprenant en outre un second convertisseur au niveau binaire (11, 103) configuré pour exécuter une conversion au niveau binaire avant ledit traitement de ladite image d'entrée dudit visage par ledit extracteur de bord (2, 103, 104 B).

**5.** Un dispositif selon la revendication 4, dans lequel ledit second convertisseur au niveau binaire (11, 103) est connecté audit extracteur de bord (2, 103, 104 B) et est configuré pour recevoir une sortie produite par ledit moyen de détermination de région de caractéristique faciale, diviser l'une de la dite pluralité de régions de recherche de caractéristique faciale en une pluralité de sous-régions plus petites, et déterminer une valeur de seuil de conversion au niveau binaire pour chacune de ladite pluralité de sous-régions plus petites à partir de la distribution de luminosité de chacune de ladite pluralité de sous-régions plus petites.

**6.** Un dispositif selon la revendication 1, ledit dispositif comprenant en outre :

un sélecteur d'adoption/rejet (9, 103) pour obtenir les positions escomptées de ladite pluralité de caractéristiques faciales, qui obtient et détermine une exactitude des positions de ladite pluralité de caractéristiques faciales, et qui rejette toute caractéristique faciale extraite par erreur avant le traitement de ladite image d'entrée dudit visage par ledit extracteur de bord (2, 103, 104 B).

**7.** Un dispositif selon la revendication 1, dans lequel ledit extracteur de bord (2, 103, 104 B) comprend un calculateur utilisant un opérateur Sobel qui produit des vecteurs gradients pour des pixels respectifs de ladite image dudit visage, lesdits vecteurs gradients ayant les grandeurs et directions indicatives desdites caractéristiques faciales.

**8.** Un dispositif selon la revendication 1, dans lequel un calcul arithmétique exécuté par ledit processeur arithmétique d'image (5, 103, 106) est un produit intérieur entre les vecteurs gradients comparés de ladite image bordée convertie au niveau binaire dudit visage et la donnée de forme respective.

**9.** Un dispositif selon la revendication 8, dans lequel un emplacement d'au moins une de la dite pluralité de régions de recherche de caractéristique faciale est déterminé par un emplacement sur ladite image dudit visage en lequel ladite valeur de corrélation est la plus grande.

**10.** Un dispositif selon la revendication 3, dans lequel : lesdits emplacements respectifs desdites autres régions de ladite pluralité de régions de recherche de caractéristique faciale sont déterminés par rapport à une position de ladite région desdits iris dudit visage.

**11.** Un dispositif selon la revendication 1, dans lequel ledit modificateur de base de données de formes (6, 103) modifie lesdites données copiées par un procédé comprenant les étapes de :

(1) déplacer ladite donnée de coordonnées de ±1 dans une direction dudit vecteur gradient,
(2) changer ladite direction dudit vecteur gradient après ladite étape de déplacer ladite donnée de coordonnées, de sorte à correspondre à ladite donnée de forme en ladite donnée de coordonnée déplacée,
(3) répéter les étapes (1) et (2) en déplaçant ladite de donnée de coordonnées de ±1 supplémentaire, poursuivant de ce fait une augmentation de valeur de ladite valeur de corrélation, et
(4) arrêter ladite étape de répétition lorsque ladite valeur de ladite valeur de corrélation n'augmente plus ou lorsque ladite valeur de la dite valeur de corrélation dépasse une valeur de seuil prédéterminée.

**12.** Un appareil selon la revendication 1, dans lequel ladite donnée de forme comprend :

lesdites données de coordonnées qui sont définies comme étant : $P_k = (l_k, m_k)$, où $l_k$, $m_k$, sont respectivement les coordonnées x, y de la kième position en laquelle ladite donnée de forme est fournie, et
lesdits vecteurs gradients sont définis comme étant :

$$v_k = (v_x, v_y),$$

où

$$v_x^2 + v_y^2 = 1$$

et

$$1 < k < n$$

(où n est un nombre de positions).

**13.** Un appareil selon la revendication 2, dans lequel ladite donnée de forme comprend :

lesdites données de coordonnées qui sont définies comme étant : $P_k = (l_k, m_k)$, où $l_k$, $m_k$, sont respectivement les coordonnées x, y de la kième position en laquelle ladite donnée de forme est fournie, et
lesdits vecteurs gradients sont définis comme étant :

$$v_k = (v_x, v_y),$$

où

$$v_x^2 + v_y^2 = 1$$

et

$$1 < k < n$$

(où n est un nombre de positions).

FIG.1

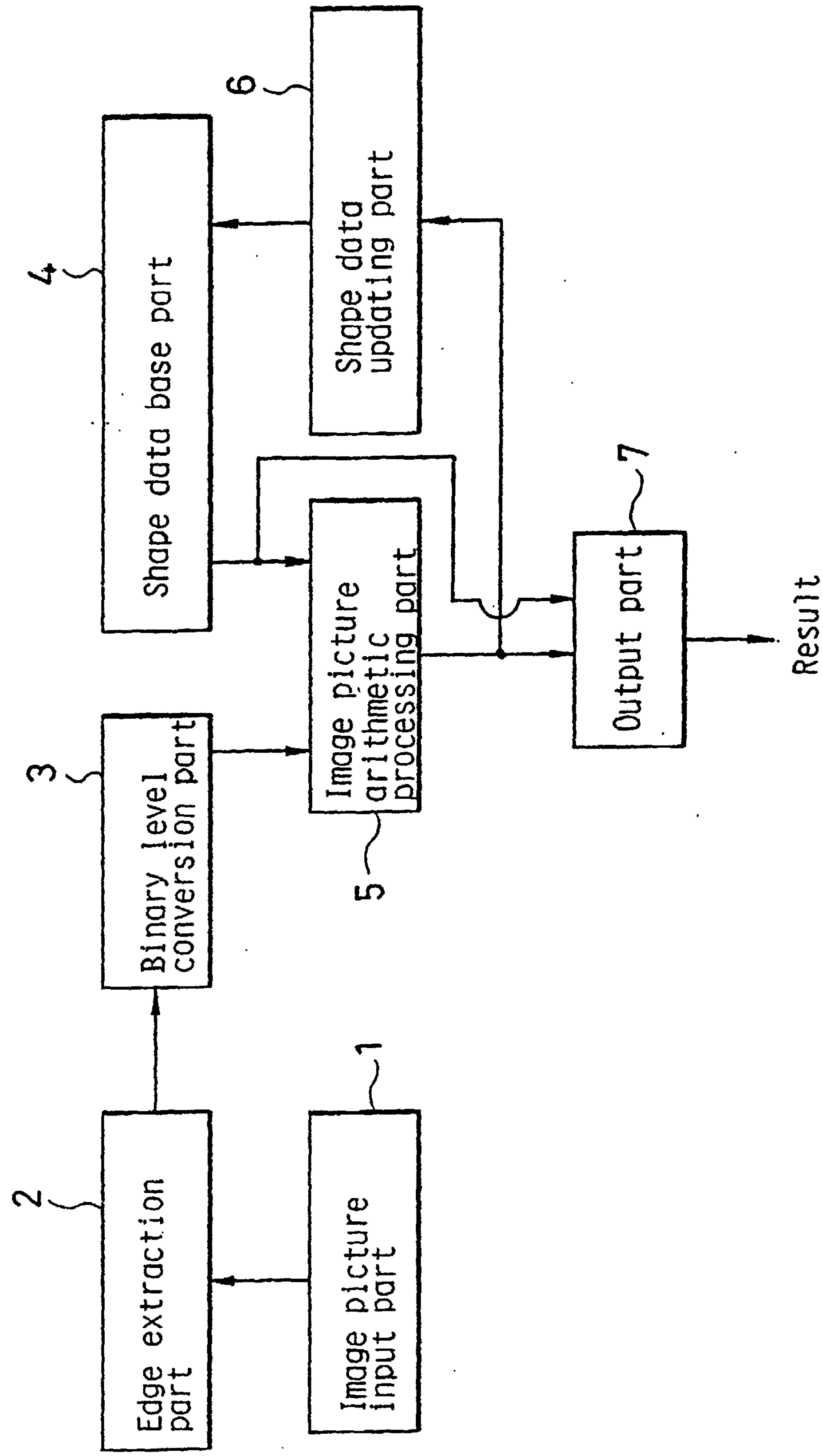

Edge extraction part
2
Image picture input part
1
Binary level conversion part
3
Shape data base part
4
Image picture arithmetic processing part
5
Shape data updating part
6
Output part
7
Result

FIG. 2

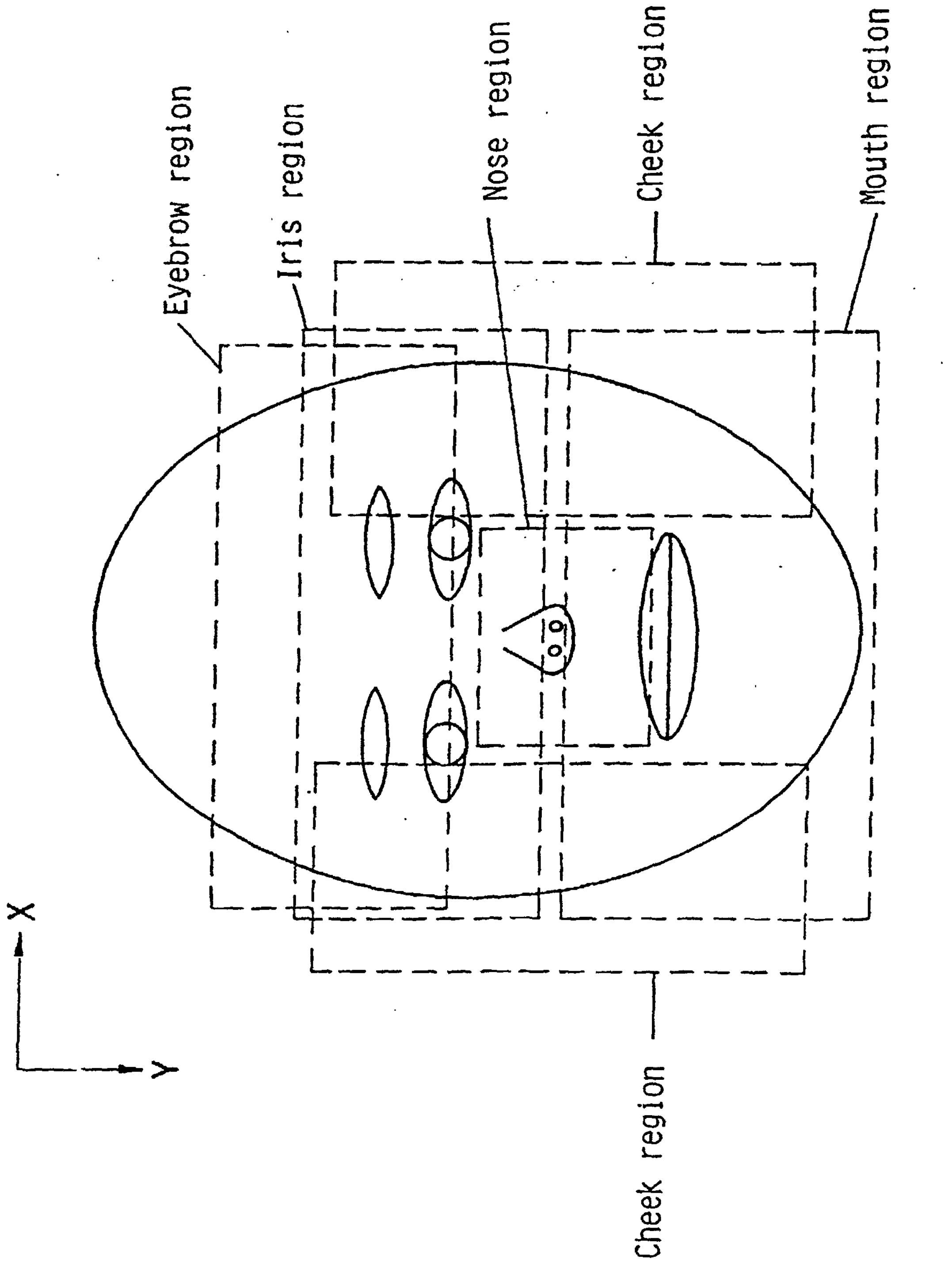

Eyebrow region
Iris region
Nose region
Cheek region
Mouth region
Cheek region
X
Y

FIG.3

| Position number k | lk | mk | Vx | Vy |
|---|---|---|---|---|
| 1 | 6 | 12 | 0 | −1 |
| 2 | 9 | 11 | −0.5 | −0.8661 |
| 3 | 11 | 9 | −0.8661 | −0.5 |
| 4 | 12 | 6 | −1 | 0 |
| 5 | 11 | 3 | −0.8661 | 0.5 |
| 6 | 9 | 1 | −0.5 | 0.8661 |
| 7 | 6 | 0 | 0 | 1 |
| 8 | 3 | 1 | 0.5 | 0.8661 |
| 9 | 1 | 3 | 0.8661 | 0.5 |
| 10 | 0 | 6 | 1 | 0 |
| 11 | 1 | 9 | 0.8661 | 0.5 |
| 12 | 3 | 11 | 0.5 | 0.8661 |

FIG. 4

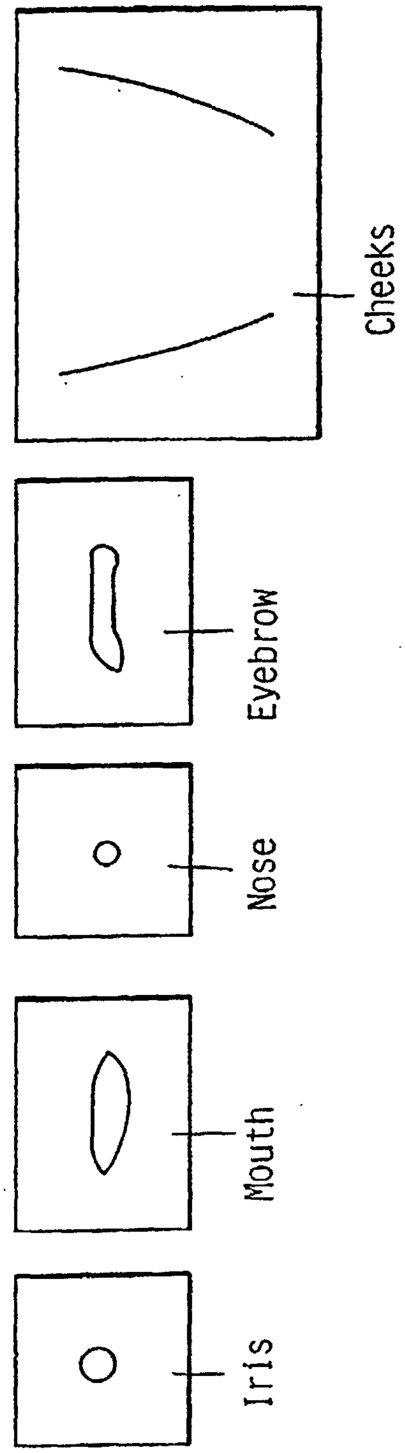

FIG. 5

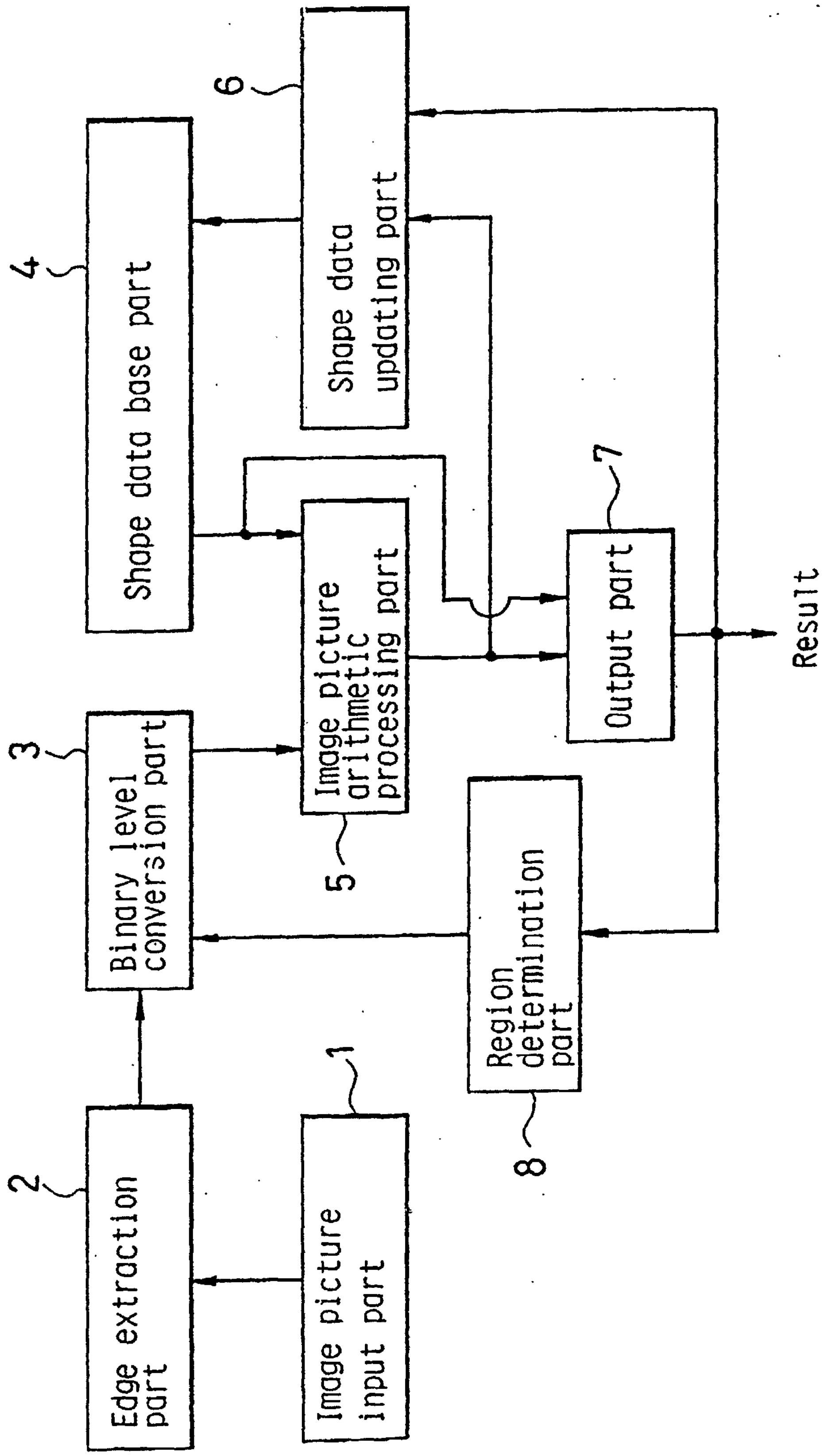
Image picture input part
1
Edge extraction part
2
Binary level conversion part
3
Shape data base part
4
Image picture arithmetic processing part
5
Shape data updating part
6
Output part
7
Result
Region determination part
8

FIG. 6

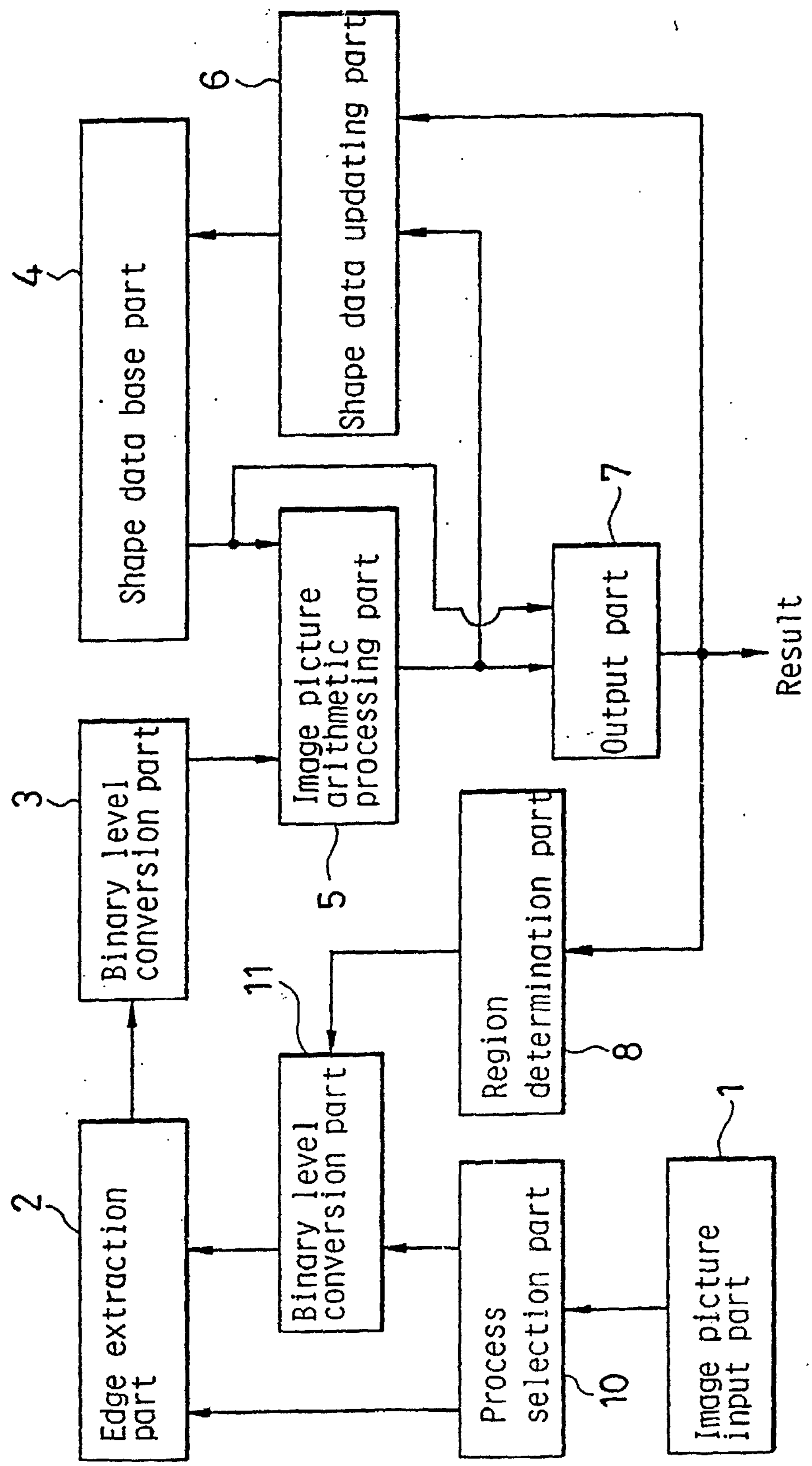
Image picture input part
1
Process selection part
10
Binary level conversion part
11
Edge extraction part
2
Binary level conversion part
3
Image picture arithmetic processing part
5
Shape data base part
4
Shape data updating part
6
Output part
7
Result
Region determination part
8

FIG. 7

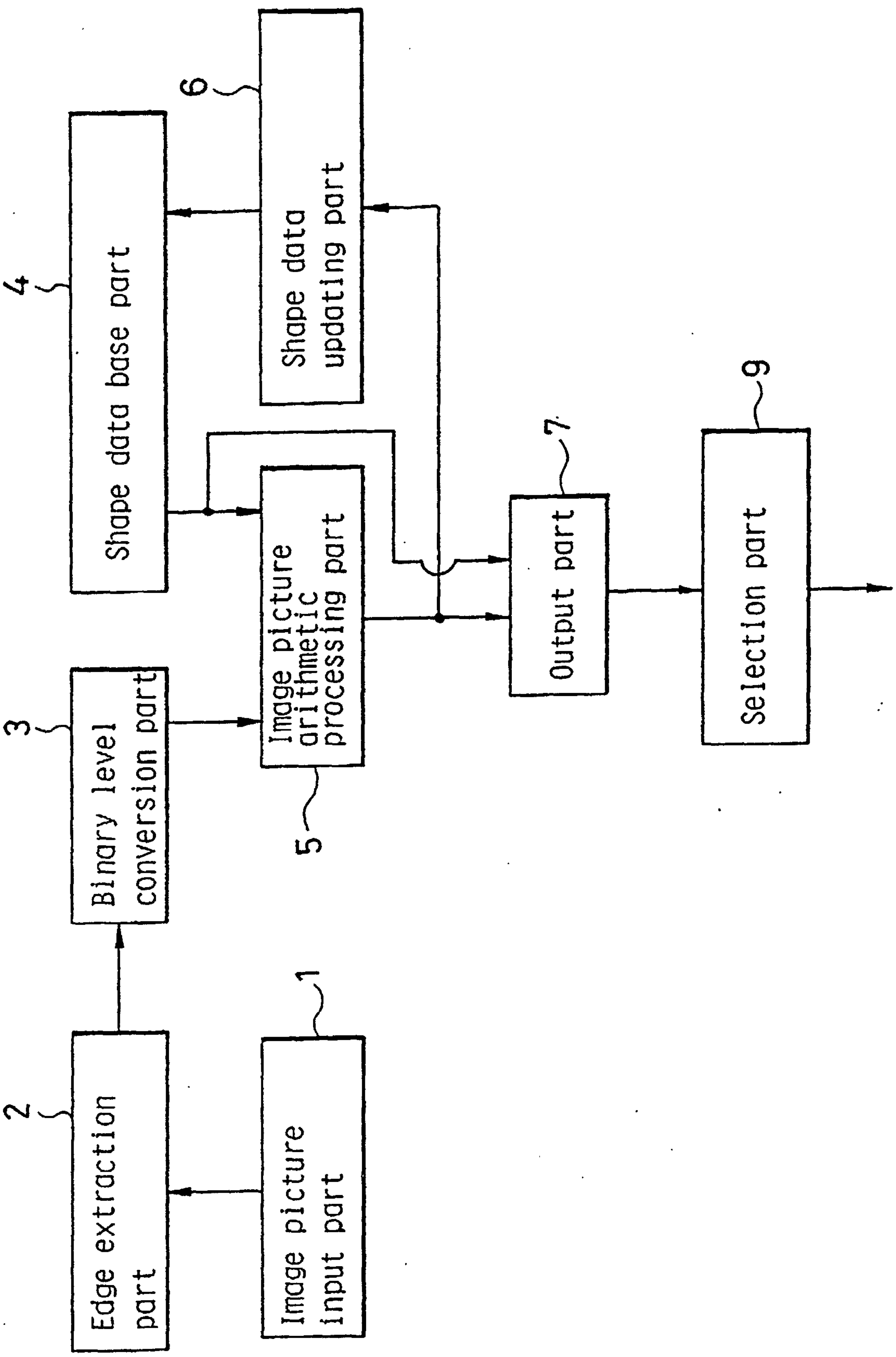

Edge extraction part
2
Image picture input part
1
Binary level conversion part
3
Shape data base part
4
Image picture arithmetic processing part
5
Shape data updating part
6
Output part
7
Selection part
9

FIG.8

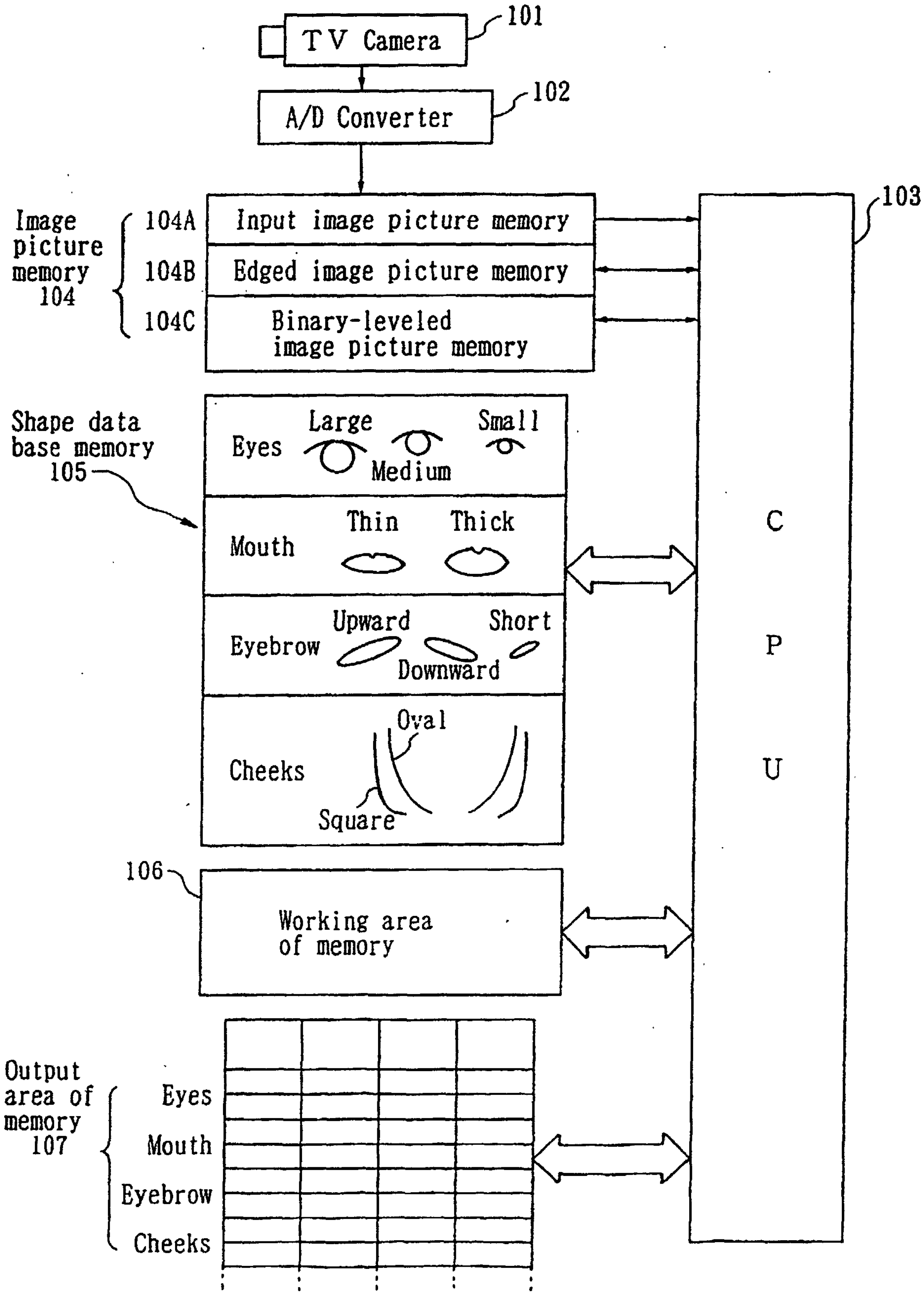

TV Camera
101
A/D Converter
102
103
Image picture memory 104
104A
Input image picture memory
104B
Edged image picture memory
104C
Binary-leveled image picture memory
Shape data base memory 105
Eyes
Large
Medium
Small
Mouth
Thin
Thick
Eyebrow
Upward
Downward
Short
Cheeks
Oval
Square
106
Working area of memory
Output area of memory 107
Eyes
Mouth
Eyebrow
Cheeks
C
P
U

FIG. 9(a)

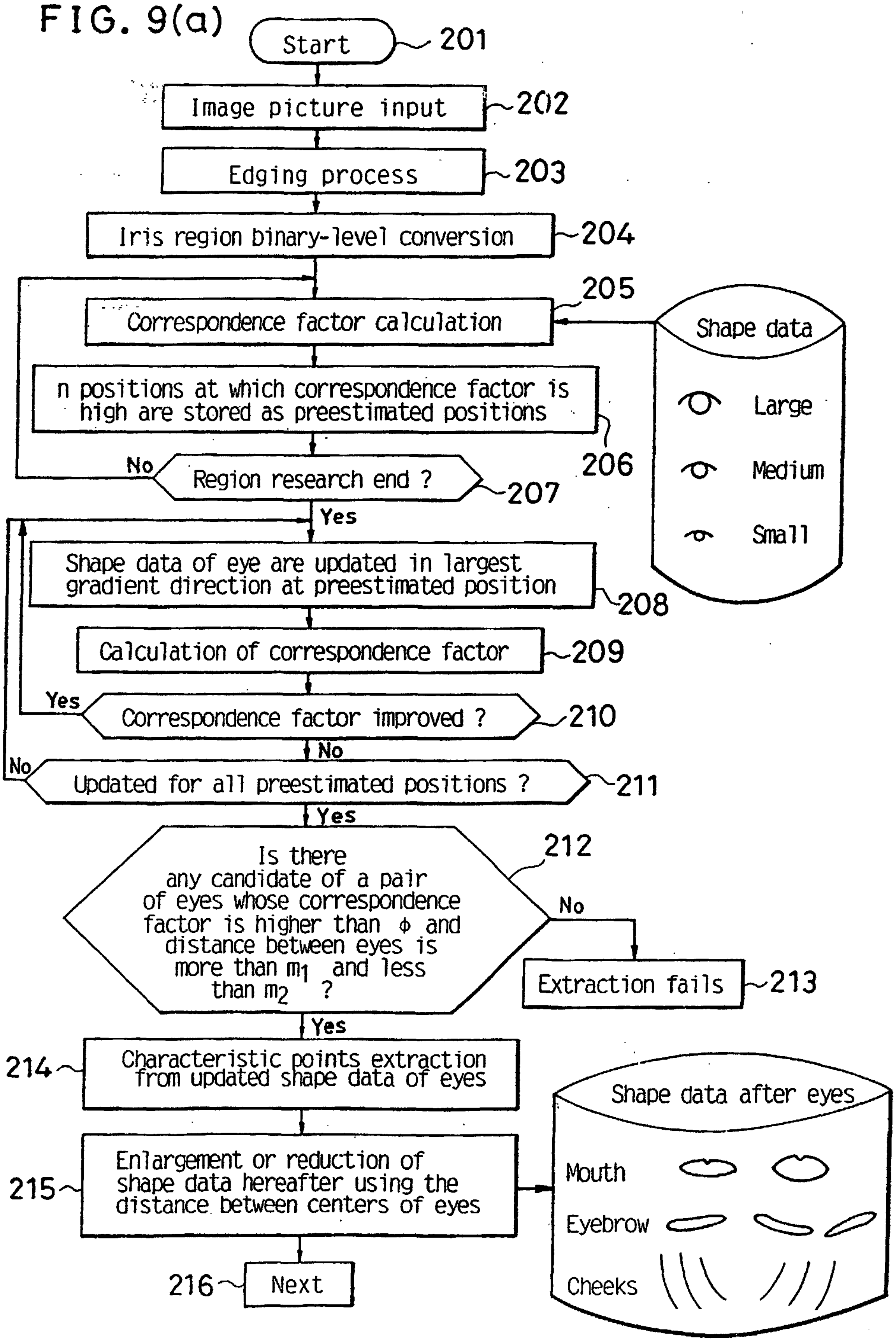

Start
201
Image picture input
202
Edging process
203
Iris region binary-level conversion
204
Correspondence factor calculation
205
Shape data
Large
Medium
Small
n positions at which correspondence factor is high are stored as preestimated positions
206
No
Region research end ?
207
Yes
Shape data of eye are updated in largest gradient direction at preestimated position
208
Calculation of correspondence factor
209
Yes
Correspondence factor improved ?
210
No
No
Updated for all preestimated positions ?
211
Yes
Is there any candidate of a pair of eyes whose correspondence factor is higher than ϕ and distance between eyes is more than $m_1$ and less than $m_2$ ?
212
No
Extraction fails
213
Yes
214
Characteristic points extraction from updated shape data of eyes
215
Enlargement or reduction of shape data hereafter using the distance between centers of eyes
Shape data after eyes
Mouth
Eyebrow
Cheeks
216
Next

FIG.9(b)

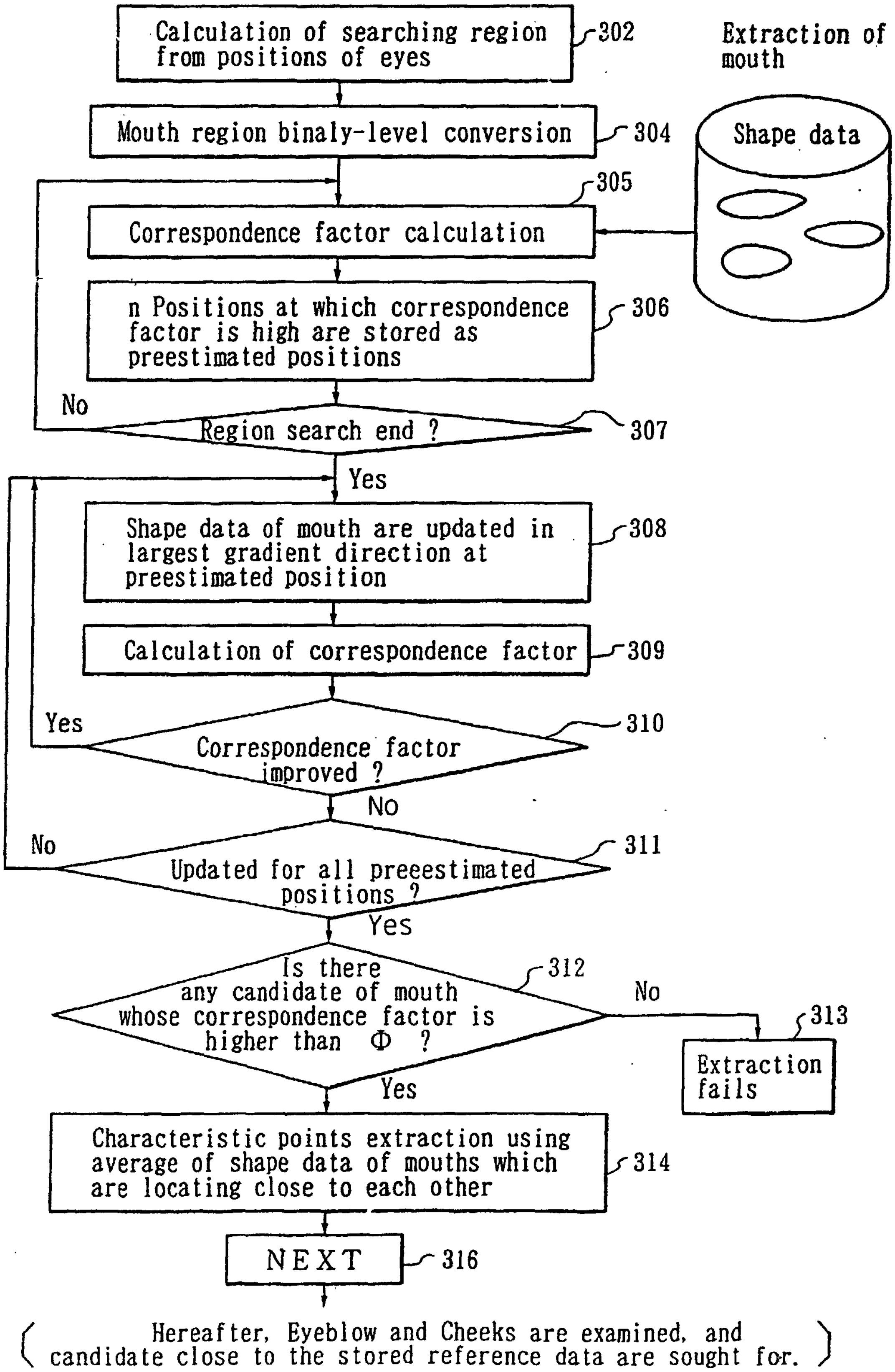
Extraction of mouth
Calculation of searching region from positions of eyes
302
Mouth region binaly-level conversion
304
Shape data
305
Correspondence factor calculation
n Positions at which correspondence factor is high are stored as preestimated positions
306
No
Region search end ?
307
Yes
Shape data of mouth are updated in largest gradient direction at preestimated position
308
Calculation of correspondence factor
309
Yes
Correspondence factor improved ?
310
No
No
Updated for all preeestimated positions ?
311
Yes
Is there any candidate of mouth whose correspondence factor is higher than Φ ?
312
No
Extraction fails
313
Yes
Characteristic points extraction using average of shape data of mouths which are locating close to each other
314
NEXT
316
( Hereafter, Eyeblow and Cheeks are examined, and candidate close to the stored reference data are sought for. )